(19) Europäisches Patentamt
European Patent Office
Office européen des brevets

(11) Publication number: 0 275 700
A1

(12) # EUROPEAN PATENT APPLICATION

(21) Application number: 87311356.7

(22) Date of filing: 23.12.87

(51) Int. Cl.4 C07C 51/64 , C07C 53/42

(30) Priority: 29.12.86 US 947061

(43) Date of publication of application:
27.07.88 Bulletin 88/30

(84) Designated Contracting States:
AT BE CH DE ES FR GB GR IT LI LU NL SE

(71) Applicant: Stauffer Specialty and Food Products Company, Inc.
300 South Riverside Plaza
Chicago Illinois 60606(US)

(72) Inventor: Wilkes, Karl A.
5613 Fresno Avenue
Richmond California 94804(US)

(74) Representative: Smith, Sydney et al
Elkington and Fife High Holborn House 52/54
High Holborn
London WC1V 6SH(GB)

(54) **Process for reducing color in fatty acid chlorides.**

(57) A process for reducing the color of fatty acid chlorides produced by a phosgenation process which comprises contacting the fatty acid chloride with a strong oxidizer for a period of time sufficient to reduce the Gardner number by from about 2 to about 8 units is disclosed and claimed.

EP 0 275 700 A1

# PROCESS FOR REDUCING COLOR IN FATTY ACID CHLORIDES

## Field of the Invention

A process for reducing color values of fatty acid chlorides prepared by phosgenation processes is disclosed. These fatty acid chlorides primarily are used in the preparation of sizing emulsions for the paper industry.

## Background of the Invention

Sizing emulsions used in the paper manufacturing industry are generally prepared by reacting a fatty acid chloride, such as stearoyl chloride, with a tertiary amine to form a diketene which is used as a sizing agent for the preparation of paper and paperboard. Many applications in industry require or call for extremely white paper or paperboard and any color which is introduced to the paper making process is carried over to the ultimate product. Preparation of acid chlorides such as stearoyl chloride by the phosgenation of fatty acids generally results in highly colored fatty acid chlorides having a Gardner number of approximately 6 to 18. It is desirable to reduce this Gardner number as low as possible to limit the impact of the fatty acid chlorides on the color of the ultimate paper produced.

## Brief Description of the Invention

A process for reducing the color value or Gardner number of a fatty acid chloride produced by phosgenation of fatty acids has been discovered which comprises contacting the fatty acid chloride with chlorine ozone or other strong oxidizer for a short period of time. The chlorine, ozone or other strong oxidizer is taken up by the fatty acid chloride and the Gardner number is normally improved by from about 2 to about 8 units.

## Detailed Description of the Invention

A process has been discovered which reduces the colorization or color values as described by Gardner number of acid chlorides produced from fatty acids by the phosgenation process similar to the following reaction.

$$CH_3(CH_2)_n\overset{O}{\overset{\|}{C}}\text{-OH} + COCl_2 \xrightarrow[80\text{-}125^\circ C]{\text{catalyst}} CH_3(CH_2)_n\overset{O}{\overset{\|}{C}}\text{-Cl} + HCl + CO_2$$

where n = 10 to 20.

The color reducing process comprises contacting the fatty acid chloride with chlorine, ozone or other strong oxidizer for a period of time sufficient to allow the chlorine, ozone or other strong oxidizer to react with the fatty acid chloride and/or by-products and other impurities and reduce the Gardner number from about 2 to about 8 units.

This process is effective on fatty acid chlorides having a carbon atom chain from $C_{12}$ to $C_{22}$ and is especially useful on those having a carbon atom chain of 12 to 18. Among the specific fatty acid chlorides for which this process is especially effective are stearoyl chloride, lauroyl chloride and palmitoyl chloride.

The amount of chlorine, ozone or other strong oxidizer necessary to react with the fatty acid chloride and/or by-products and other impurities and the length of time over which the reaction can take place are of course dependent upon each other. The more oxidizer that is available in general the shorter the reaction time and the less oxidizer that is available provided the longer the reaction time. It is preferred that the amount of oxidizer range from about 0.2 mole % to about 10 mole % of the fatty acid chloride to be decolorized and the amount of time required for the reaction range from about 0.1 to about 8 hours. The

preferred amount of oxidizer can range from about 0.4 mole % to about 8 mole % and the preferred amount of time ranges from about 0.1 to about 2 hours. The temperature of the reaction can range from about 20°C to about 125°C, preferably from about 20°C to about 80°C.

The term "contacting" means the length of time which the oxidizer is added to the reaction mixture.

Examples of strong oxidizers in addition to chlorine and ozone are calcium hypochlorite, nitrous oxide, bromine, fluorine and potassium permanganate.

The term "stearoyl chloride" refers to stearoyl chloride made from any of the commercially available grades of stearic acid which are actually mixtures of several fatty acids, including both saturated and unsaturated fatty acids.

Examples of two commercially available stearic acids, made by Emery Industries, having differing percentages of fatty acids (FA) are as follows:

| FA carbon chain length | Emersol® 132 | Emersol® 421 |
|---|---|---|
| $C_{14}$ | 2.5 | 2.0 |
| $C_{15}$ | 0.5 | 0.5 |
| $C_{16}$ | 50.0 | 26.5 |
| $C_{17}$ | 1.5 | 2.0 |
| $C_{18}$ | 45.5 | 68.0 |
| $C_{18}'$ | – | 1.0 |

Other commercially named fatty acids such as lauric acid similarly contain differing percentages of fatty acids, the major percentage of which is at or near the carbon chain length of the formal fatty acid name given the commercial mixture.

## EXAMPLES

The following examples show embodiments of this invention which were conducted by the following procedures.

### EXAMPLE 1

A 350 g sample of stearoyl chloride prepared from a tallow fatty acid was sparged subsurface with chlorine in a 1 2-liter stirred flask at a rate of 1.8 weight % chlorine per hour for one hour at 20°C. A slight heat rise was noted upon chlorine addition. Gases from the flask were sent to a caustic scrubber. During the contacting period the Gardner color of the stearoyl chloride changed as follows:

TABLE 1

| Time (mins.) | Gardner Color No. |
|---|---|
| 0 | 14 |
| 15 | 14 |
| 30 | 8 |
| 45 | 8 |
| 60 | 8 |

EXAMPLE 2

A 350 g sample of stearoyl chloride prepared from a tallow fatty acid was sparged subsurface with chlorine in a 1 2 liter stirred flask at a rate of 0.7 weight % chlorine per hour for one hour at 60°C. A slight heat rise was noted upon chlorine addition. Gases from the flask were sent to a caustic scrubber. During the contacting period the Gardner color of the stearoyl chloride changed as follows:

TABLE 2

| Time (mins.) | Gardner Color No. |
|---|---|
| 0 | 14 |
| 15 | 10 |
| 30 | 9 |
| 45 | 8 |
| 60 | 8 |

EXAMPLE 3

A 350 g sample of stearoyl chloride prepared from a tallow fatty acid was sparged subsurface with chlorine in a 1 2 liter stirred flask at a rate of 5.1 weight % chlorine per hour for one hour at 60°C. A slight heat rise was noted upon chlorine addition. Gases from the flask were sent to a caustic scrubber. During the contacting period the Gardner color of the stearoyl chloride changed as follows:

TABLE 3

| Time (mins.) | Gardner Color No. |
|---|---|
| 0 | 14 |
| 15 | 13 |
| 30 | 12 |
| 45 | 10 |
| 60 | 10 |

EXAMPLE 4

A 350 g sample of stearoyl chloride prepared from a tallow fatty acid was sparged subsurface with chlorine in a 1 2-liter stirred flask at a rate of 1.8 weight % chlorine per hour for one hour at 60°C. A slight heat rise was noted upon chlorine addition. Gases from the flask were sent to a caustic scrubber. During the contacting period the Gardner color of the stearoyl chloride changed as follows:

TABLE 4

| Time (mins.) | Gardner Color No. |
|---|---|
| 0 | 14 |
| 15 | 10 |
| 30 | 9 |
| 45 | 8 |
| 60 | 8 |

EXAMPLE 5

Lauroyl chloride which was prepared from a coconut based fatty acid was decolorized using chlorine. In a 300-gallon stirred reactor, approximately 1600 pounds of lauroyl chloride was sparged subsurface with 0.1 weight % chlorine per hour for one hour. The Gardner color subsequently changed from 12 to 6 during the 1-hour contacting period. The lauroyl chloride was then stripped of any excess chlorine.

EXAMPLE 6

Approximately 1200 g of stearoyl chloride were made in a 2-liter stirred flask by reaction with phosgene and a catalyst at 100°C. At the end of the reaction step with the phosgene and catalyst still present, a sample of the reaction mixture was purified and found to have a Gardner color of 7. Chlorine was added subsurface to the stearoyl chloride at 100°C at a rate of 0.003 g chlorine/gram stearoyl chloride for one hour. At the end of the chlorination step the color was found to have changed from Gardner 7 to 4. The decolorized stearoyl chloride product was then stripped of any remaining chlorine, phosgene, and other gases prior to further processing.

EXAMPLE 7

Approximately 1500 lb of stearoyl chloride was made in a stirred, 300-gallon reactor by reacting stearic acid with phosgene and a catalyst at 100°C. After stripping, cooling and filtering the product, a sample of the product showed a color of Gardner 13. The stearoyl chloride was then contacted with a mixture of 1.8 lb ozone/day as approximately 1% ozone in air at 60°C for a period of 8 hours such that 0.0004 lbs of ozone was added per pound of product (0.24 m% ozone). After the 8-hour contacting period, the color of the stearoyl chloride had changed from Gardner 13 to 6.

EXAMPLE 8

A 500 g sample of stearoyl chloride prepared from a tallow fatty acid was sparged subsurface in an unstirred 1-liter bottle with approxi mately 1.3 g ozone/hour as approximately 1% ozone in air at 20°C for 1 hour (1.6 mole % more ozone added). Gases from the bottle were scrubbed in a 2% potassium iodide solution. Approximately 0.001 g ozone/g stearoyl chloride was added. During the contacting period the Gardner color changed from 10 to 3.

EXAMPLE 9

A 500 g sample of stearoyl chloride prepared from a tallow fatty acid was sparged subsurface in an unstirred 1-liter bottle with approxi mately 1.6 g ozone hour as approximately 1% ozone in air at 100°C for one hour (1.9 mole % ozone). Gases from the bottle were scrubbed with a 2% potassium iodide solution to determine the ozone uptake. Approximately 0.002 g ozone g steroyl chloride (1.2 mole % ozone) was taken up by the steroyl chloride. The Gardner color changed from 10 to 3.

## Claims

1. A process for decolorizing fatty acid chlorides having a carbon chain of from 12 to 22 comprising contacting the fatty acid chloride with a strong oxidizer for a period of time sufficient to reduce the Gardner number from about 2 to about 8 units.

2. The process of Claim 1 wherein the strong oxidizer is selected from the group consisting of chlorine. ozone, bromine, fluorine, potassium permanganate and nitrous oxide.

3. The process of Claim 2 wherein the strong oxidizer is chlorine or ozone.

4. The process of Claim 3 wherein the amount of oxidizer used to contact the fatty acid ranges from 0.1 mole % to 10 mole %.

5. The process of Claim 3 wherein the amount of contact time ranges from about 0.1 to about 8 hours.

6. The process of Claim 3 wherein the temperature of the reaction ranges from about 20°C to about 125°C.

7. The process of Claim 3 wherein the fatty acid chloride contains from 12 to 18 carbon atoms.

8. The process of Claim 3 wherein the amount of oxidizer used to contact the fatty acid ranges from about 0.4 mole % to about 8 mole %.

9. The process of Claim 3 wherein the amount of contact time ranges from about 0.1 to about 2.

10. The process of Claim 3 wherein the temperature of the reaction ranges from about 20°C to about 80°C.

European Patent Office

# EUROPEAN SEARCH REPORT

Application Number

EP 87 31 1356

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 002 808 (HOECHST)<br>* claim * | 1-3 | C 07 C 51/64<br>C 07 C 53/42 |
| A | US-A-4 182 728 (DES MARAIS)<br>* column 1, lines 51-57, claims * | 1-3 | |
| A | PATENT ABSTRACTS OF JAPAN, vol. 8, no. 77 (C-218)[1514], 10th April 1984; & JP - A - 59 1442 (NIPPON POLYURETHANE) 06-01-1984 | 1 | |
| A | US-A-2 748 151 (HICKMAN)<br>* claims 1-3 * | 1-3 | |
| A | US-A-1 936 739 (TOWNEND)<br>* claims * | 1-3 | |

TECHNICAL FIELDS SEARCHED (Int. Cl.4)

C 07 C 51/00
C 07 C 53/00

The present search report has been drawn up for all claims

| Place of search | Date of completion of the search | Examiner |
|---|---|---|
| BERLIN | 23-03-1988 | PROBERT C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document